(19) Europäisches Patentamt
European Patent Office
Office européen des brevets

(11) Veröffentlichungsnummer: **0 094 536**
**B1**

(12) **EUROPÄISCHE PATENTSCHRIFT**

(45) Veröffentlichungstag der Patentschrift:
26.11.86

(21) Anmeldenummer: 83104254.4

(22) Anmeldetag: 30.04.83

(51) Int. Cl.⁴: **C 08 G 63/06,** C 08 J 3/12,
C 08 J 9/28, C 08 G 63/70

(54) **Mikroporöse, pulverförmige Polylactide und Verfahren zu deren Herstellung.**

(30) Priorität: 14.05.82 DE 3218151

(43) Veröffentlichungstag der Anmeldung:
23.11.83 Patentblatt 83/47

(45) Bekanntmachung des Hinweises auf die Patenterteilung:
26.11.86 Patentblatt 86/48

(84) Benannte Vertragsstaaten:
CH FR GB LI NL

(56) Entgegenhaltungen:
US-A-3 607 793
US-A-4 247 498

Chemical Abstracts Band 93, Nr. 26, 29. Dezember
1980, Columbus, Ohio, USA B. KALB et al. "General
crystallisation behavior of poly(L-lactic acid)",
Seite 9, Spalte 1, Abstract Nr. 240075s
Chemical Abstracts Band 99, Nr. 8, 22. August 1983,
Columbus, Ohio, USA S. GOGOLEWSKI et al.
"Resorbable materials of poly(L-lactide). III.
Porous materials for medical application", Seite
311, Spalte 1, Abstract Nr. 58873x
Polymer 21, (1980), 607-612

(73) Patentinhaber: Akzo GmbH, Postfach 10 01 49
Kasinostrasse 19- 23, D-5600 Wuppertal- 1 (DE)

(72) Erfinder: Lange, Wolfgang, Dr. Dipl.- Chem.,
Rosenstrasse 12, D-8753 Obernburg (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft mikroporöse, pulverförmige Polylactide und Verfahren zu deren Herstellung, die mit Wirkstoffen beladen werden können und die in der Lage sind, dieselben über einen längeren Zeitraum wieder abzugeben.

Es ist bekannt, Polylactide in verschiedenen Lösungsmitteln unter Zusatz von Porenbildnern zu lösen und die Lösung zu Formkörpern zu verarbeiten. Rieselfähige, mikroporöse, für die Aufnahme von Wirkstoffen geeignete Pulver entstehen dabei nicht.

Löst man Polylactide in Benzol oder Toluol und kühlt die Lösung unter Verformen ab, so erhält man kompakte durchsichtige Folien.

In der US-PS 3 607 793 wird die Herstellung von porösen polymeren Folien beschrieben,wobei man das Polymer in einem organischen Lösungsmittel löst bei erhöhten Temperaturen und die erhaltene Lösung abkühlt, ein Gel daraus bildet und die organische Flüssigkeit extrahiert. Die Herstellung von Pulvern, insbesondere von porösen Polylactidpulvern wird in dieser Schrift nicht erwähnt.

Auch in der US-PS 4 247 498 wird lediglich die Herstellung von porösen Filmen und porösen Blocken beschrieben. Es wird dort weder die Herstellung von Pulvern noch die Herstellung von pulverförmigen, porösen Polylactiden erwähnt.

In der Zeitschrift Polymer 21, (1980), 607-612 wird über das Kristallisationsverhalten von Polylactiden berichtet. So wird die Gewinnung von Einkristallen aus sehr verdünnten Lösungen in Xylol erwähnt. Löst man Polylactide in Chloroform, m-Cresol, Isoamylalkohol oder einem Gemisch aus Äthylenglykol und Aceton und bringt unter Rühren dieses Gemisch in Koagulationsflüssigkeiten, so erhält man poröse faserartige Produkte. Die Herstellung von mikroporösen, pulverförmigen Polylactiden wird in diesem Fachaufsatz an keiner Stelle erwähnt.

Es besteht deshalb noch ein Bedürfnis nach einem verbesserten, wirtschaftlich arbeitenden Verfahren zur Herstellung von mikroporösen Polylactidpulvern mit verbesserten Eigenschaften.

Aufgabe der Erfindung ist somit die Herstellung von mikroporösen rieselfähigen Polylactidpulvern, die geeignet sind, größere Mengen an Wirkstoffen wie Medikamente, Polypeptide, Hormone u.dgl., Pestizide, Spurenelemente, Duftstoffe etc. zu speichern und über längere Zeit hinaus wieder abzugeben.

Aufgabe sind ferner Polylactidpulver, die mit festen oder flüssigen Wirkstoffen beladen, in einer Polymermatrix verteilt werden können.

Diese Aufgabe wird durch ein Verfahren gemäß Anspruch 1 gelöst. Weitere vorteilhafte Ausführungsformen werden in den Ansprüchen 2 bis 6 wiedergegeben.

Gegenstand der Erfindung sind ferner mikroporöse pulverförmige Polylactide gemäß Anspruch 7.

Die Erfindung wird durch folgendes Beispiel näher erläutert:

## Beispiel

90,3 g d(-)-Polylactid mit einem Molekulargewicht von ca. 40 000 wurden in 400 ml Xylol gelost und anschließend ohne Rühren abkühlen gelassen. Die dabei erhaltene, homogen erscheinende Masse wurde abgenutscht und anschließend bei 60°C vakuumgetrocknet. Hg-Intrusionsmessungen an diesem rieselfähigen Pulver ergaben einen Wert von 55 % Porenvolumen.

## Patentansprüche

1. Verfahren zur Herstellung von mikroporösen pulverförmigen Polylactiden, dadurch gekennzeichnet, daß man ein Polylactid durch Erwärmen in Xylol löst, die entstandene klare Lösung abkühlt und das Xylol entfernt.

2. Verfahren nach Anspruch 1, dadurch gekennzeichnet, daß man das Polylactid in der Siedehitze löst.

3. Verfahren nach den Ansprüchen 1 bis 2, dadurch gekennzeichnet, daß man das Xylol durch Absaugen entfernt.

4. Verfahren nach den Ansprüchen 1 bis 3, dadurch gekennzeichnet, daß als Polylactid Polymere der dl-Milchsäure, l(+)-Milchsäure, d(-)-Milchsäure allein oder in Kombination verwendet werden.

5. Verfahren nach den Ansprüchen 1 bis 4, dadurch gekennzeichnet, daß man Copolymere der Milchsäuren und weiterer Hydroxycarbonsäuren verwendet.

6. Verfahren nach Anspruch 5, dadurch gekennzeichnet, daß man als weitere Hydroxycarbonsäure Glykolsäure verwendet.

7. Mikroporöse, pulverförmige Polylactide, erhältlich nach einem Verfahren gemäß den Ansprüchen 1 bis 6.

## Claims

1. A process for the production of microporous powderform polylactides, characterized in that a polylactide is dissolved in xylene by heating, the clear solution formed is cooled and the xylene is removed.

2. A process as claimed in Claim 1, characterized in that the polylactide is dissolved in boiling heat.

3. A process as claimed in Claims 1 and 2, characterized in that the xylene is removed by filtration under suction.

4. A process as claimed in Claims 1 to 3, characterized in that polymers of dl-lactic acid, l(+)-lactic acid, d(-)-lactic acid either

**0 094 536**

individually or in combination are used as the polylactide.

5. A process as claimed in Claims 1 to 4, characterized in that copolymers of lactic acids and other hydroxycarboxylic acids are used.

6. A process as claimed in Claim 5, characterized in that glycolic acid is used as the other hydroxycarboxylic acid.

7. Microporous powder-form polylactides obtainable by the process claimed in Claims 1 to 6.

**Revendications**

1. Procédé de préparation de polylactides sous forme pulvérulente et microporeuse, caractérisé en ce que l'on dissout un polylactide par chauffage dans du xylène, on refroidit la solution limpide obtenue, et on élimine le xylène.

2. Procédé conforme à la revendication 1, caractérisé en ce que l'on dissout le polylactide à la température d'ébullition.

3. Procédé conforme aux renvendications, 1 et 2, caractérisé en ce que l'on élimine le xylène par aspiration sous vide.

4. Procédé conforme aux revendications 1 à 3, caractérisé en ce que l'on utilise comme polylactide des polymères de l'acide dl-lactique, de l'acide 1 (+) -lactique, de l'acide d(-)-lactique, seuls ou en combinaison.

5. Procédé conforme aux revendications 1 à 4, caractérisé en ce que l'on utilise des copolymères des acides lactiques et d'autres acides hydroxycarboxyliques.

6. Procédé conforme à la revendication S, caractérisé en ce que l'on utilise l'acide glycolique comme autre acide hydroxycarboxylique.

7. Polylactide sous forme pulvérulente et microporeuse, obtenu selon un procédé conforme aux revendications 1 à 6.